# FASCICULE DE BREVET EUROPEEN

(11) **EP 1 276 526 B1**
(45) Date de publication et mention de la délivrance du brevet: **29.06.2005**
(21) Numéro de dépôt: 01903972.6
(22) Date de dépôt: 26.01.2001
(51) Int. Cl.: A61M 5/30, A61M 5/303

(54) **SERINGUE SANS AIGUILLE MUNIE D'UN OPERCULE CONTENANT LE PRINCIPE ACTIF**
NADELLOSE SPRITZE MIT EINER DEN WIRKSTOFF BEINHALTENDEN ABDECKUNG
NEEDLELESS SYRINGE PROVIDED WITH AN INNER SEAL CONTAINING THE ACTIVE PRINCIPLE

(30) Priorité: 02.02.2000 FR 0001311
(43) Date de publication de la demande: 22.01.2003
(73) Titulaire: CROSSJECT, 75181 Paris Cedex 04 (FR)
(72) Inventeur: ALEXANDRE, Patrick, F-70100 Gray (FR); BAUD, Georges, F-83260 La Crau (FR); GAUTIER, Philippe, F-91220 Le Plessis Paté (FR); ROLLER, Denis, F-91590 La Ferté Alais (FR)
(86) Numéro de dépôt international: PCT/FR2001/000251
(87) Numéro de publication internationale: WO 2001/056637

(56) Documents cités:
- WO-A-96/12513
- WO-A-96/25190
- US-A- 4 124 024
- US-A- 5 204 253
- US-A- 5 899 880

## Description

Le domaine technique de l'invention est celui des seringues sans aiguille utilisées pour les injections hypodermiques ou intramusculaires de divers principes actifs pulvérulents, à usage thérapeutique pour la médecine humaine ou vétérinaire.

Plus spécifiquement, l'invention se rapporte à une seringue sans aiguille mettant en oeuvre un générateur de gaz destiné à créer une onde de pression pour éjecter les particules de principe actif. Un opercule claquable, placé sur le chemin des gaz, permet d'obtenir le niveau de pression seuil permettant d'éjecter les particules avec une vitesse suffisamment élevée. En fait, la libération soudaine des gaz crée dans la seringue une onde de choc et c'est ce choc qui va porter et accélérer les particules afin de les expulser. La spécificité de l'invention réside dans le fait que l'opercule a une autre fonction que celle de contribuer à créer une onde de choc : il sert également de système de retenue des particules solides de principe actif.

Les seringues sans aiguille fonctionnant par génération d'un choc pour injecter les particules solides de principe actif existent déjà et ont fait l'objet de plusieurs brevets. On peut citer en particulier le brevet WO 94/24263 qui décrit une seringue sans aiguille fonctionnant par libération d'une réserve de gaz pour entraîner les particules solides de principe actif. Dans ce brevet, l'une des caractéristiques principales est que les particules sont maintenues en permanence sur le chemin des gaz, entre deux membranes soufflables.

Une seringue sans aiguille selon le préambule de la revendication 1 est connue du document US-A-5204253.

D'autres brevets concernant des dispositifs autres que des seringues sans aiguille décrivent des mécanismes d'éjection de particules solides. On peut citer par exemple le brevet US 5 478 744 se rapportant à un dispositif qui permet de bombarder des cultures cellulaires avec des particules inertes ou biologiquement actives, et dont le principe de fonctionnement repose sur la libération d'un gaz sous pression dans un tube qui peut être alimenté latéralement par des particules. Ce même brevet décrit un mécanisme d'éjection de particules solides faisant intervenir une masselotte portant lesdites particules, et mise en vitesse sous l'effet des gaz sous pression, puis brutalement stoppée par une butée laissant les particules poursuivre leur course à vitesse élevée.

Aucun des deux brevets cités ci-avant ne se rapporte à des mécanismes d'éjection de particules solides impliquant un opercule ayant la double fonction de porter les particules et de créer une onde de choc.

La seringue sans aiguille selon l'invention est apte à éjecter des particules solides de principe actif par l'effet d'une onde de choc produit par la libération d'un gaz sous pression.

L'utilisation d'une charge pyrotechnique permet de répondre à la double exigence de performance et de fiabilité. En effet, une charge de poudre peut produire une grande quantité de gaz sur un temps très court et donc contribuer à générer une onde de choc dont la vitesse du front peut être très élevée. Par conséquent, les particules de principe actif subissent une accélération importante, favorisant leur aptitude à la pénétration, tandis que le souffle en sortie de seringue se dissipe très rapidement dans l'air ambiant. En outre, la mise à feu de charges pyrotechniques miniaturisées est parfaitement maîtrisée au moyen d'initiateurs largement éprouvés et adaptés aux caractéristiques desdites charges à initier, conférant ainsi aux seringues sans aiguille selon l'invention un caractère de grande fiabilité. Enfin, l'utilisation d'un opercule assurant la double fonction de contenir les particules de principe actif et de créer une onde de choc, permet de réduire l'encombrement de la seringue et de simplifier le mécanisme de fonctionnement en réduisant le nombre de pièces impliquées.

L'objet de la présente invention concerne une seringue sans aiguille comprenant successivement un générateur de gaz, une chambre d'expansion des gaz, un moyen de retenue des particules de principe actif et un tube d'éjection desdites particules, caractérisée en ce que le moyen de retenue des particules est constitué par un opercule possédant au moins une cavité destinée à loger les particules, ledit opercule étant conçu pour se rompre au niveau de cette cavité lorsqu'il est soumis à un niveau de pression seuil. Préférentiellement, l'opercule présente au niveau de sa cavité, au moins une ligne d'affaiblissement destinée à favoriser son ouverture en libérant les particules. Selon un mode de réalisation préféré de l'invention, l'opercule est constitué d'au moins deux éléments symétriques accolés selon une ligne d'ouverture permettant la libération des particules. L'un des buts à atteindre avec ce type de configuration, est la cassure nette de l'opercule pour libérer proprement les particules solides de principe actif, sans qu'il y ait production de particules parasites provenant de l'opercule susceptibles d'être expulsées. De plus, la libération des particules de principe actif devant s'effectuer juste avant la création de l'onde de choc dans le tube, ou au plus tard en même temps, il est donc souhaitable de favoriser une rupture de l'opercule au niveau de la cavité contenant les particules de principe actif. De façon avantageuse, l'opercule a une position transversale par rapport à l'axe du tube d'éjection et présente sur sa face aval une avancée centrale dans laquelle est située la cavité.

Selon un mode de réalisation, l'opercule est fixe et comporte en son centre une cavité fermée. Pour cette configuration, l'opercule marque la frontière entre la chambre d'expansion et le tube d'éjection. De cette manière, lorsque la pression dans la chambre d'expansion atteint une valeur seuil, l'opercule s'ouvre au niveau de sa cavité, permettant de libérer les particules de principe actif dans le tube d'éjection, tout en provoquant, de façon quasi-simultanée, la création d'une onde de choc dans ledit tube.

Selon un mode de réalisation préféré de l'invention, l'opercule constitue l'une des deux extrémités d'un cylindre creux situé dans la chambre d'expansion. Plus précisément, la paroi latérale externe du cylindre creux est au contact de la paroi latérale interne de la chambre, et l'opercule est situé à l'extrémité du cylindre la plus éloignée du générateur de gaz. De façon avantageuse, la cavité de l'opercule est borgne et est obturée par une membrane transversale fixée dans le tube d'éjection. Préférentiellement, la membrane transversale est fixée au niveau d'un épaulement interne marquant la limite entre la chambre et le tube d'éjection et le cylindre creux est maintenu dans la chambre grâce à la membrane dont il est au contact. Avantageusement, l'opercule est collé à la membrane transversale. De façon préférentielle, les gaz émis par le générateur de gaz entraînent le déplacement du cylindre creux jusqu'à l'épaulement interne contre lequel il vient en butée, ce déplacement impliquant la rupture de la membrane par le passage de l'avancée centrale de l'opercule, et provoquant aussitôt la libération des particules solides de principe actif. De façon avantageuse, la membrane est de faible épaisseur et est constituée par un matériau inélastique. La membrane a une double fonction : elle obture la cavité pour maintenir les particules dans leur logement et sert également de surface de fixation pour le cylindre creux. Préférentiellement, le rebord de la cavité borgne est effilé pour faciliter l'ouverture de la membrane, ladite membrane étant prédécoupée radialement jusqu'au centre pour favoriser une ouverture en pétales. Cette membrane doit être dimensionnée de manière à ce qu'elle n'entrave pas le déplacement du cylindre creux en se montrant trop résistante, et qu'elle ne se divise pas en plusieurs morceaux au moment de sa rupture. Selon ce second mode de réalisation préféré de l'invention, les gaz émis provoquent d'abord le déplacement du cylindre creux qui déchire la membrane et permet la libération des particules de principe actif dans le tube d'éjection. Ensuite le cylindre vient se bloquer contre l'épaulement interne, et, lorsque la pression des gaz atteint une valeur seuil dans l'espace compris entre le générateur de gaz et l'opercule, ledit opercule s'ouvre en créant une onde de choc.

Avantageusement, l'opercule est calibré pour céder à une pression d'au moins 20 bars, mais cette pression de rupture doit être adaptée en fonction de la granulométrie et de la densité des particules de principe actif, de manière à ce que la vitesse d'éjection des particules à la sortie du tube d'éjection soit supérieure à 750 mètres par seconde.

Selon un autre mode de réalisation de l'invention, l'opercule possède plusieurs cavités alignées suivant une même direction. De façon préférentielle, l'opercule présente une ligne d'affaiblissement rectiligne traversant toutes les cavités. Par ce biais, toutes les cavités s'ouvrent de façon concommittente permettant la création d'une seule onde de choc intense.

De façon avantageuse, le générateur de gaz est un générateur de gaz pyrotechnique comprenant une charge pyrotechnique et un dispositif d'initiation de ladite charge.

préférentiellement, le dispositif d'initiation comprend un système de percussion et une amorce couramment utilisés en industrie pyrotechnique. Mais il est également possible d'initier la charge pyrotechnique par d'autres moyens, et notamment, ceux impliquant soit un cristal piézo-électrique, soit un rugueux ou même une pile. Avantageusement, le déclencheur est situé à l'une des extrémités de la seringue sous la forme d'un bouton poussoir, pour faciliter sa préhension et son actionnement.

Les seringues sans aiguille selon l'invention bénéficient des avantages liés à un fonctionnement par onde de choc, en particulier en terme de vitesse d'éjection des particules, tout en assurant un maintien fiable des particules en mode stockage.

De plus, l'utilisation d'un opercule ayant la double fonction de loger les particules de principe actif et de créer une onde de choc permet de simplifier le mécanisme de fonctionnement de la seringue en limitant le nombre de pièces impliquées.

Enfin, le maintien des particules dans le chemin des gaz est une garantie pour toutes les particules de principe actif de se retrouver en position d'être accélérées par l'onde de choc.

On donne ci-après la description détaillée de deux modes de réalisation de seringues sans aiguilles en se référant aux figures 1 à 4.
La figure 1 est une vue en coupe axiale longitudinale d'une seringue sans aiguille selon l'invention possédant un opercule comprenant une cavité obturée par une membrane transversale, ladite seringue n'ayant pas encore fonctionné.
La figure 2 est une vue en coupe axiale longitudinale agrandie du système de retenue des particules de la seringue de la figure 1, après avoir fonctionné.
La figure 3 est une vue en coupe axiale longitudinale d'une seringue sans aiguille possédant un opercule portant une cavité fermée, ladite seringue n'ayant pas encore fonctionné.
La figure 4 est une vue en coupe axiale longitudinale agrandie du système de retenue des particules de la seringue de la figure 3 après avoir fonctionné.

Les termes « aval » et « amont » dans la description détaillée ci-après sont utilisés par rapport au sens de propagation des gaz dans la seringue.

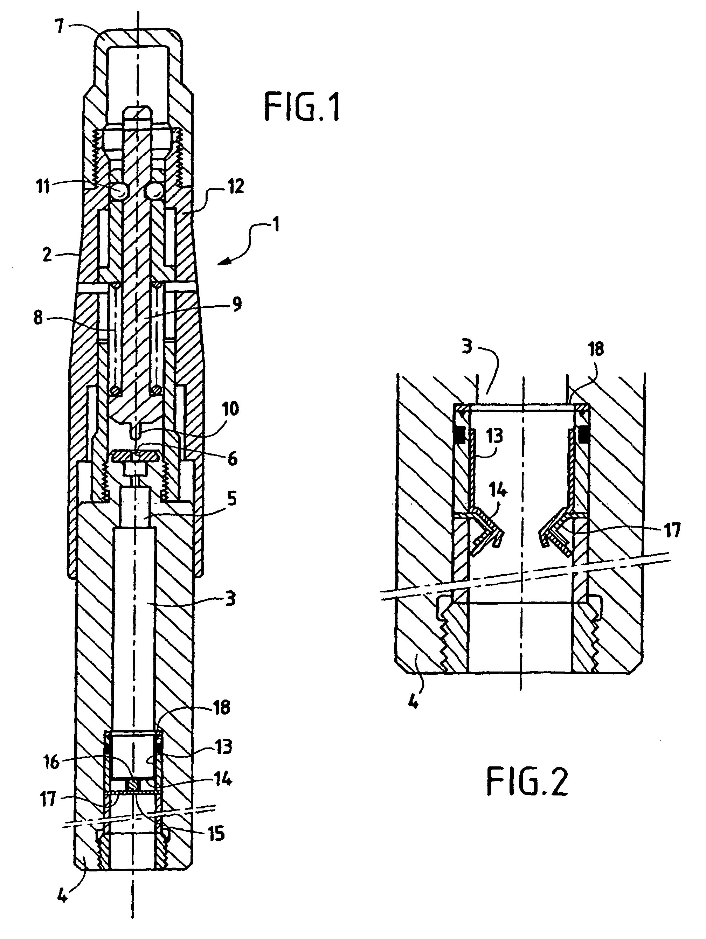

En se référant à la figure 1, une seringue sans aiguille 1, selon un mode de réalisation préféré de l'invention, comprend successivement un générateur de gaz pyrotechnique 2, une chambre d'expansion 3, un système de maintien des particules et un tube d'éjection 4 desdites particules destiné à venir en appui contre la peau du patient à traiter.

Le générateur pyrotechnique 2 de gaz comprend un dispositif d'initiation d'une charge pyrotechnique 5 faisant intervenir un dispositif de percussion et une amorce 6. Le dispositif de percussion qui est déclenché par un bouton poussoir 7 comprend un ressort 8 et une masselotte 9 allongée munie d'un percuteur 10. La masselotte 9 est bloquée par au moins une bille 11 de maintien coincée entre ladite masselotte 9 et un corps cylindrique creux 12 dans lequel est susceptible de se déplacer ladite masselotte 9. L'amorce 6 et la charge pyrotechnique 5, de forme sensiblement cylindrique, sont logées dans le corps cylindrique creux 12, en aval de la masselotte 9. La charge pyrotechnique 5, qui est logée dans le corps creux 12, débouche sur un espace libre de la seringue qui constitue la chambre d'expansion 3 des gaz qui seront issus de la combustion de la charge pyrotechnique 5.

La chambre 3 est délimitée à son extrémité opposée à celle constituée par la charge pyrotechnique 5 par une pièce cylindrique creuse 13 dont l'une des extrémités est libre et l'autre est fermée par un opercule 14 susceptible de se rompre au delà d'un niveau de pression seuil dans ladite chambre 3.

De façon plus précise, c'est l'opercule 14 qui délimite la longueur de la chambre 3.

La pièce cylindrique creuse 13 est positionnée dans la seringue de manière à ce que son extrémité fermée par l'opercule 14 soit en aval de son extrémité libre. L'opercule 14 se présente sous la forme d'une pièce circulaire plane ayant une face aval au centre de laquelle est disposée une protubérance creuse, de forme cylindrique, constituant une cavité 15 pour loger les particules solides de principe actif. La pièce circulaire plane présente en son centre, au niveau de la zone sur laquelle est située la protubérance, une ligne d'affaiblissement 16 dirigée suivant un diamètre de ladite protubérance. Avantageusement, la pièce cylindrique creuse 13 comportant l'opercule 14 est en polycarbonate. Une membrane 17 inextensible, de faible épaisseur, transversale par rapport à l'axe du tube d'éjection 4, est fixée audit tube 4. Cette membrane peut, par exemple, être constituée en polyéthylène.

La pièce 13 est placée dans la seringue 1 de sorte que, d'une part, son extrémité libre vienne en appui contre un épaulement interne 18 situé dans la chambre 3, et, d'autre part, la protubérance centrale de l'opercule 14 soit au contact de la membrane 17 transversale. Ce contact peut être assuré par un léger collage. De cette manière, la cavité 15 contenant les particules solides de principe actif se retrouve fermée par la membrane 17. Le tube d'éjection 4 peut avantageusement se terminer par un bourrelet amortissant pour faciliter le contact de la seringue 1 sur la peau du patient.

Le mode de fonctionnement de ce mode de réalisation préféré de l'invention s'effectue comme suit.

L'utilisateur positionne la seringue 1 de façon à ce que l'extrémité du tube d'éjection 4 vienne en appui contre la peau du patient à traiter.

Une pression sur le bouton poussoir 7 permet, d'une part, au corps cylindrique creux 12 de se déplacer jusqu'à ce que sa partie évasée se présente en face de la bille 11 de maintien et, d'autre part, de comprimer le ressort 8. La bille 11 sort de son logement, libérant alors la masselotte 9, qui, soumise à l'action du ressort 8 qui se détend est brutalement accélérée vers l'amorce 6, le percuteur 10 en avant. La réaction de l'amorce 6 entraîne la mise à feu de la charge pyrotechnique 5 qui se décompose en émettant des gaz, lesdits gaz envahissant instantanément la chambre 3 d'expansion et l'intérieur de la pièce cylindrique creuse 13. La pression alors générée dans ladite chambre 3 provoque le déplacement de ladite pièce cylindrique creuse 13 qui déchire la membrane 17 avec le bord de la cavité 15, permettant la libération des particules solides de principe actif dans le tube d'éjection 4. Comme le montre la figure 2, la pièce cylindrique creuse 13 finit sa course lorsque la partie circulaire plane de l'opercule 14 vient en butée contre le bord périphérique de la membrane 17 non affecté par le passage de la protubérance et faisant saillie dans le tube 4. Lorsque la pièce cylindrique creuse 13 se retrouve ainsi bloquée en bout de course, le niveau de pression croit dans la chambre 3, ainsi que dans la pièce cylindrique 13, jusqu'à entraîner l'ouverture de l'opercule 14 au niveau de sa ligne d'affaiblissement 16. Cette ouverture crée dans le tube 4, une onde de choc qui emporte et accélère les particules encore sous la forme d'un nuage diffus. La libération des particules dans le tube 4 et l'ouverture de l'opercule 14 doivent s'effectuer sur un intervalle de temps très court, n'excédant pas quelques millisecondes pour que les particules n'aient pas eu le temps de se regrouper entre elles par inertie.

Selon un autre mode de réalisation, la seringue sans aiguille comprend successivement un générateur de gaz pyrotechnique 102, une chambre d'expansion 103, un système de maintien des particules et un tube d'éjection 104 desdites particules destiné à venir en appui contre la peau du patient à traiter. Le générateur de gaz pyrotechnique 102 qui fonctionne à partir d'un dispositif de percussion, une charge pyrotechnique 105 et une amorce 106, est en tout point identique à celui décrit ci-avant concernant le mode de réalisation préféré de l'invention.

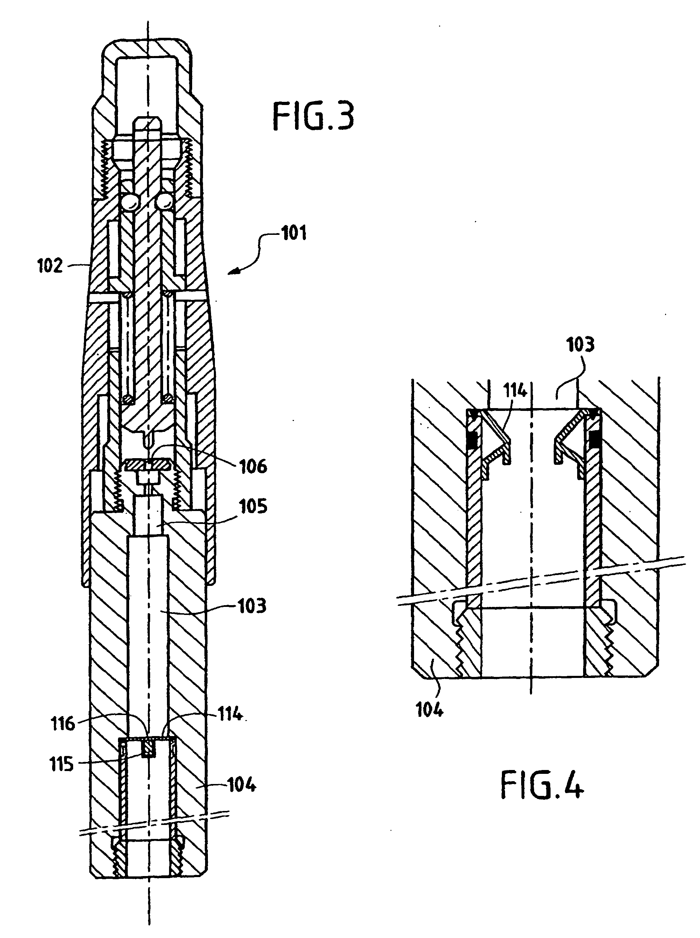

En se référant à la figure 3, la chambre d'expansion 103 a une forme sensiblement cylindrique et son diamètre est inférieur à celui du tube d'éjection 104, également cylindrique et qui la prolonge. Un épaulement interne marque cette différence de diamètres. La chambre 103 est délimitée, suivant son axe, d'une part, par une face dans laquelle débouche la charge pyrotechnique 105 et, d'autre part, par un opercule 114 susceptible de se rompre au delà d'un niveau de pression seuil dans ladite chambre 103. L'opercule 114 se présente sous la forme d'une pièce circulaire plane ayant une face aval au centre de laquelle est disposée une protubérance creuse de forme cylindrique constituant une cavité 115 pour loger les particules solides de principe actif. Ladite pièce circulaire présente en son centre, au niveau de la zone sur laquelle est située la protubérance, une ligne d'affaiblissement 116 dirigée suivant un diamètre de ladite protubérance. Cette cavité 115 est obturée par une membrane légère de façon à retenir les particules. L'opercule 114 se retrouve en position transversale par rapport à l'axe de la chambre 103 et du tube 104. Cet opercule, qui est pincé entre l'épaulement interne marquant la frontière entre la chambre 103 et le tube 104, et une pièce venant en appui contre ledit épaulement, est inamovible. Le tube d'éjection 104 peut avantageusement se terminer par un bourrelet amortissant pour faciliter le contact de la seringue sur la peau du patient.

Le mode de fonctionnement de ce mode de réalisation se déroule comme suit. L'étape qui permet d'aboutir à la mise à feu de la charge pyrotechnique 105 par l'utilisateur est rigoureusement identique à celle décrite précédemment pour le premier mode de réalisation préféré de l'invention. Les gaz émis par la combustion de la charge pyrotechnique 105 envahissent la chambre 103. La pression dans ladite chambre 103 croît et finit par ouvrir l'opercule 114 au niveau de sa ligne d'affaiblissement 116, entraînant, simultanément, la libération des particules de principe actif dans le tube 104 et la création d'une onde de choc qui emporte et accélère lesdites particules encore sous la forme d'un nuage diffus vers la peau du patient à traiter. L'opercule 114, une fois ouvert, vient se plaquer contre la paroi latérale interne du tube d'éjection 104.

## Revendications

1. Seringue sans aiguille (1) comprenant successivement un générateur de gaz (2), une chambre (3) d'expansion des gaz, un opercule (14) possédant au moins une cavité (15) destinée à loger des particules de principe actif, ledit opercule (14) étant conçu pour se rompre au niveau de cette cavité (15) lorsqu'il est soumis à un niveau de pression seuil, et un tube d'éjection (4) desdites particules, **caractérisée en ce que** l'opercule (14) constitue l'une des deux extrémités d'un cylindre creux (13) situé dans la chambre d'expansion (3) et susceptible de se déplacer sous l'effet des gaz produits par le générateur (2), la cavité (15) dudit opercule (14) étant borgne et obturée par une membrane (17) transversale fixée dans le tube d'éjection (4).

2. Seringue sans aiguille selon la revendication 1, **caractérisée en ce que** l'opercule (14) présente, au niveau de sa cavité (15), une ligne d'affaiblissement (16) destinée à favoriser son ouverture en libérant les particules.

3. Seringue sans aiguille selon la revendication 1, **caractérisée en ce que** l'opercule (14) est constitué d'au moins deux éléments symétriques accolés selon une ligne d'ouverture permettant la libération des particules.

4. Seringue sans aiguille selon la revendication 1, **caractérisée en ce que** l'opercule (14) a une position transversale par rapport à l'axe du tube d'éjection (4), et présente sur sa face aval, qui est extérieure au cylindre (13), une avancée centrale dans laquelle est située la cavité (15).

5. Seringue sans aiguille selon la revendication 1, **caractérisée en ce que** la membrane (17) transversale est fixée au niveau d'un épaulement interne marquant la limite entre la chambre (3) et le tube d'éjection (4) et le cylindre creux (13), qui est maintenu dans la chambre (3) grâce à la membrane (17) dont il est au contact, peut venir en butée contre ledit épaulement sous l'effet des gaz libérés par le générateur (2).

6. Seringue sans aiguille selon l'une quelconque des revendications 1 ou 6, **caractérisée en ce que** le rebord de la cavité borgne (15) est effilé pour faciliter l'ouverture de la membrane (17), ladite membrane (17) étant prédécoupée radialement jusqu'au centre pour favoriser une ouverture en pétales.

7. Seringue sans aiguille selon la revendication 1, **caractérisée en ce que** l'opercule (14) est calibré pour céder à une pression d'au moins 20 bars.

8. Seringue sans aiguille selon la revendication 1, **caractérisée en ce que** le générateur de gaz (2) est un générateur de gaz pyrotechnique comprenant une charge pyrotechnique (5) et un dispositif d'initiation.

9. Seringue sans aiguille selon la revendication 8, **caractérisée en ce que** le dispositif d'initiation comprend un système de percussion et une amorce (6).

## Patentansprüche

1. Nadellose Spritze (1), die hintereinander aufweist: einen Gasgenerator (2), einen Expansionsraum (3) für die Gase, eine Abdeckung (14), die mindestens einen Hohlraum (15) besitzt, der dazu bestimmt ist, Wirkstoffteilchen aufzunehmen, wobei die Abdeckung (14) so ausgebildet ist, dass sie in Höhe dieses Hohlraums (15) reißt, wenn sie einem Druckpegelschwellwert ausgesetzt wird, und ein Ausstoßrohr (4) für die Teilchen, **dadurch gekennzeichnet, dass** die Abdeckung (14) eines der beiden Enden eines Hohlzylinders (13) bildet, der sich im Expansionsraum (3) befindet und sich unter der Wirkung der vom Generator (2) erzeugten Gase verschieben kann, wobei der Hohlraum (15) der Abdeckung (14) ein Sackloch ist und von einer Quermembran (17) verschlossen wird, die im Ausstoßrohr (4) befestigt ist.

2. Nadellose Spritze nach Anspruch 1, **dadurch gekennzeichnet, dass** die Abdeckung (14) in Höhe ihres Hohlraums (15) eine Sollbruchlinie (16) aufweist, die dazu bestimmt ist, ihre Öffnung zum Freisetzen der Teilchen zu begünstigen.

3. Nadellose Spritze nach Anspruch 1, **dadurch gekennzeichnet, dass** die Abdeckung (14) aus mindestens zwei symmetrischen Elementen besteht, die entlang einer Öffnungslinie aneinander liegen, die das Freisetzen der Teilchen erlaubt.

4. Nadellose Spritze nach Anspruch 1, **dadurch gekennzeichnet, dass** die Abdeckung (14) eine Querposition bezüglich der Achse des Ausstoßrohrs (4) hat und auf ihrer stromabwärts hinten liegenden Fläche, die außerhalb des Zylinders (13) liegt, eine zentrale Auskragung aufweist, in der sich der Hohlraum (15) befindet.

5. Nadellose Spritze nach Anspruch 1, **dadurch gekennzeichnet, dass** die Quermembran (17) in Höhe einer inneren Schulter befestigt ist, die die Grenze zwischen dem Raum (3) und dem Ausstoßrohr (4) darstellt, und der Hohlzylinder (13), der im Raum (3) mit Hilfe der Membran (17) gehalten wird, mit der er in Kontakt steht, unter der Wirkung der vom Generator (2) freigesetzten Gase gegen die Schulter in Anschlag gelangen kann.

6. Nadellose Spritze nach einem der Ansprüche 1 oder 5, **dadurch gekennzeichnet, dass** die Randleiste des sacklochförmigen Hohlraums (15) sich verjüngt, um die Öffnung der Membran (17) zu erleichtern, wobei die Membran (17) radial bis in die Mitte vorausgeschnitten ist, um eine blütenblattförmige Öffnung zu ermöglichen.

7. Nadellose Spritze nach Anspruch 1, **dadurch gekennzeichnet, dass** die Abdeckung (14) bemessen ist, um einem Druck von mindestens 20 Bar nachzugeben.

8. Nadellose Spritze nach Anspruch 1, **dadurch gekennzeichnet, dass** der Gasgenerator (2) ein pyrotechnischer Gasgenerator ist, der eine pyrotechnische Ladung (5) und eine Zündvorrichtung aufweist.

9. Nadellose Spritze nach Anspruch 8, **dadurch gekennzeichnet, dass** die Zündvorrichtung ein Schlagsystem und einen Zünder (6) aufweist.

## Claims

1. Needle-less syringe (1) comprising, in succession: a gas-generator (2); an expansion chamber (3) for the gases; a cap (14) possessing at least one cavity (15) intended to accommodate particles of active principle, the said cap (14) being designed to break at the level of the said cavity (15) when subjected to a threshold pressure level; and a tube (4) for the ejection of the said particles, **characterised in that** the cap (14) constitutes one of the two ends of a hollow cylinder (13) which is situated in the expansion chamber (3) and is capable of being displaced under the effect of the gases produced by the generator (2), the cavity (15) of the said cap (14) being blind and being stopped up by a transverse diaphragm (17) fixed in the ejection tube (4).

2. Needle-less syringe according to claim 1, **characterised in that** the cap (14) has, at the level of its cavity (15), a line of reduced thickness (16) intended to promote its opening while releasing the particles.

3. Needle-less syringe according to claim 1, **characterised in that** the cap (14) is made up of at least two symmetrical elements coupled together along a line of opening which permits the release of the particles.

4. Needle-less syringe according to claim 1, **characterised in that** the cap (14) has a transverse position in relation to the axis of the ejection tube (4) and has, on its downstream face which is outside the cylinder (13), a central projection in which the cavity (15) is situated.

5. Needle-less syringe according to claim 1, **characterised in that** the transverse diaphragm (17) is fixed at the level of an internal shoulder marking the boundary between the chamber (3) and the ejection tube (4), and the hollow cylinder (13), which is held in said chamber (3) with the aid of the diaphragm (17), with which it is in contact, is able to abut against the said shoulder under the effect of the gases released by the generator (2).

6. Needle-less syringe according to either of claims 1 or 6, **characterised in that** the rim of the blind cavity (15) is tapered in order to facilitate the opening of the diaphragm (17), the said diaphragm (17) being precut radially as far as the centre in order to promote opening into petals.

7. Needle-less syringe according to claim 1, **characterised in that** the cap (14) is calibrated so as to give way at a pressure of at least 20 bars.

8. Needle-less syringe according to claim 1, **characterised in that** the gas-generator (2) is a pyrotechnical gas-generator comprising a pyrotechnical charge (5) and an initiating device.

9. Needle-less syringe according to claim 8, **characterised in that** the initiating device comprises a percussion system and a detonator (6).
